# EUROPEAN PATENT SPECIFICATION

(11) **EP 3 450 539 B1**
(45) Date of publication and mention of the grant of the patent: **17.02.2021**
(21) Application number: 17789196.7
(22) Date of filing: 05.04.2017
(51) Int. Cl.: C12M 1/34, C12Q 1/02

(54) **EVALUATION SYSTEM FOR BIOLOGICAL SAMPLES, EVALUATION METHOD FOR BIOLOGICAL SAMPLES AND PROGRAM FOR CONTROLLING EVALUATION OF BIOLOGICAL SAMPLES**
BEWERTUNGSSYSTEM FÜR BIOLOGISCHE PROBEN, BEWERTUNGSVERFAHREN FÜR BIOLOGISCHE PROBEN UND PROGRAMM ZUR STEUERUNG DER BEWERTUNG BIOLOGISCHER PROBEN
SYSTÈME D'ÉVALUATION POUR ÉCHANTILLONS BIOLOGIQUES, PROCÉDÉ D'ÉVALUATION POUR ÉCHANTILLONS BIOLOGIQUES ET PROGRAMME DE CONTRÔLE DE L'ÉVALUATION DES ÉCHANTILLONS BIOLOGIQUES

(30) Priority: 28.04.2016 JP 2016090458
(43) Date of publication of application: 06.03.2019
(73) Proprietor: FUJIFILM Corporation, Tokyo 106-8620 (JP)
(72) Inventor: YAMAGUCHI, Naoko, Ashigarakami-gun Kanagawa 258-8538 (JP)
(74) Representative: Klunker IP Patentanwälte PartG mbB
(86) International application number: PCT/JP2017/014165
(87) International publication number: WO 2017/187908

(56) References cited:
- EP-A1- 2 418 473
- WO-A1-01/11340
- WO-A2-2008/008149
- WO-A2-2008/079243
- JP-A- S6 040 955
- JP-A- H07 505 710
- JP-A- 2003 526 772
- JP-A- 2005 095 012
- JP-A- 2014 530 358
- JP-A- 2016 510 418
- US-A1- 2005 282 268
- US-A1- 2015 215 537
- Erik Meijering ET AL: "Meijering et al. 1/32 Time-Lapse Imaging Time-Lapse Imaging", , 1 January 2008 (2008-01-01), pages 401-440, XP055544675, Retrieved from the Internet: URL:https://imagescience.org/meijering/pub lications/download/tlmi2008.pdf [retrieved on 2019-01-18]
- GRAFF, R.D. et al.: "Microplate live cell assay system for early events in mechanotransduction", Analytical Biochemistry, vol. 318, no. 2, July 2003 (2003-07), pages 181-186, XP055436389,
- GOLEBIEWSKA, U. et al.: "Measuring fast calcium fluxes in cardiomyocytes", J. Visual. Exp., vol. 57, 29 November 2011 (2011-11-29), pages 1-7, XP055436390,

## Description

### BACKGROUND OF THE INVENTION

### 1. Field of the Invention

The present invention relates to a biological sample evaluation system, a biological sample evaluation method, and a biological sample evaluation control program capable of evaluating a biological sample having undergone a predetermined process at a desired time interval.

### 2. Description of the Related Art

In the related art, in biology, a test is frequently performed in which a predetermined process is performed on biological tissue, and a nature change after the process is evaluated. Examples of the process include addition of a substance and irradiation with light, and an example of evaluation includes observation of morphology using a microscope.

Specifically, there is a case where a process such as addition of a chemical is performed on a biological sample, a temporal change of the biological sample is observed by performing evaluation (for example, capturing of an image of the biological sample) at a desired time interval from the time at which a predetermined time elapses, and thus an effect of the chemical is checked.

US 2005/282268 describes a culture microscope having an incubator chamber which controls a culture environment in which cells are cultured, an imaging optical system which photographs the cells, and a controller which controls a time-lapse photographing performed by the imaging optical system.

EP 2 418 473 A1 describes a biological specimen observation apparatus which observes temporal change in a biological specimen, comprising: macro image acquisition means for acquiring a macro image by capturing an image of a macro region of a broad range of the biological specimen, while time lapse observation is performed; biological change region extracting means for extracting a biological change region, which is a region of change in the biological specimen, from the acquired macro image; micro observation point setting means for registering a micro observation point corresponding to the extracted biological change region; micro image acquisition means for capturing an image of a micro region of change in the biological specimen identified at the micro observation point, while time lapse observation is performed; and judgment means for judging whether or not biological change has continued in the biological change region at the micro observation point, from the acquired micro image, wherein the micro observation point setting means updates the registered micro observation point on the basis of a judgment result by the judgment means.

WO 2008/079243 describes a system is provided for screening compounds for biological activity in cultures of Dictyostelium producing time-lapse videos of each cell culture.

### SUMMARY OF THE INVENTION

Here, in a case where a temporal change of a biological sample is observed as described above, in the related art, a process may be performed on a single biological sample, and evaluation may be performed at a desired time interval from the time at which a predetermined time elapses.

However, in a case where a desired time interval for evaluation is shorter than the shortest evaluation operation time required for evaluation in an evaluation apparatus, realization thereof is not possible. For example, in a case where a morphological change after a chemical is added to a biological sample is observed with a microscope of the evaluation apparatus, processes such as movement of the biological sample between apparatuses, focusing of the microscope, and adjustment of an exposure amount of the microscope are necessary between certain observation and the next observation, and a predetermined time (for example, 60 seconds) (shortest evaluation operation time) are required therebetween.

On the other hand, in a case where a chemical is toxic, a reaction after addition of the chemical may occur abruptly, and thus it is necessary to perform observation at very short time intervals in order to elucidate the mechanism. In this case, a desired time interval for observation may be longer than the shortest evaluation operation time of an evaluation apparatus, and cannot be realized in a process and an evaluation for a single biological sample.

JP1993-080057A (JP-H5-080057A) and JP2014-206381A propose a system which automatically controls procedures from a process for a biological sample to evaluation, but does not propose any control method in a case where the shortest evaluation operation time of an evaluation apparatus is longer than a desired time interval as described above.

In light of the problem, an object of the present invention is to provide a biological sample evaluation system, a biological sample evaluation method, and a biological sample evaluation control program capable of obtaining an evaluation result at a desired time interval even in a case where the shortest evaluation operation time of an evaluation apparatus is longer than the desired time interval for observation.

The above and other objects are achieved by a biological sample evaluation system as defined in claim 1, a biological sample evaluation method as defined in claim 15 and a non-transitory computer readable recording medium as defined in claim 16. Preferred embodiments are set out in the dependent claims.

According to the biological sample evaluation system, the biological sample evaluation method, and the biological sample evaluation control program according to the aspects of the present invention, processes are sequentially performed on a plurality of biological samples of an identical type from a reference time, and the plurality of biological samples are sequentially evaluated from a time point at which a preset time elapses from the reference time. In this case, a time interval of each process on each of the biological samples is set to a time interval which is equal to or more than a shortest process operation time of a processing apparatus, and a time interval of each evaluation of each of the biological samples is set to a time interval which is equal to or more than a shortest evaluation operation time of an evaluation apparatus and is obtained by adding or subtracting a desired time interval shorter than the shortest evaluation operation time to or from the time interval of each process. By setting a time interval of each process and a time interval of each evaluation as mentioned above, it is possible to obtain an evaluation result at a desired time interval even in a case where a shortest evaluation operation time of an evaluation apparatus is longer than the desired time interval for observation.

### BRIEF DESCRIPTION OF THE DRAWINGS

Fig. 1 is a block diagram illustrating a schematic configuration of a biological sample evaluation system according to an embodiment of the present invention.
Fig. 2 is a graph illustrating examples of a time interval of each process on each biological sample and a time interval of each evaluation of each biological sample.
Fig. 3 is a graph illustrating other examples of a time interval of each process on each biological sample and a time interval of each evaluation of each biological sample.
Fig. 4 is a flowchart for explaining a shortest-time operation mode.
Fig. 5 is a flowchart for explaining an any-time operation mode.
Fig. 6 is a flowchart for explaining an execution time restriction operation mode.
Fig. 7 is a flowchart for explaining an apparatus use time restriction operation mode.

### DESCRIPTION OF THE PREFERRED EMBODIMENTS

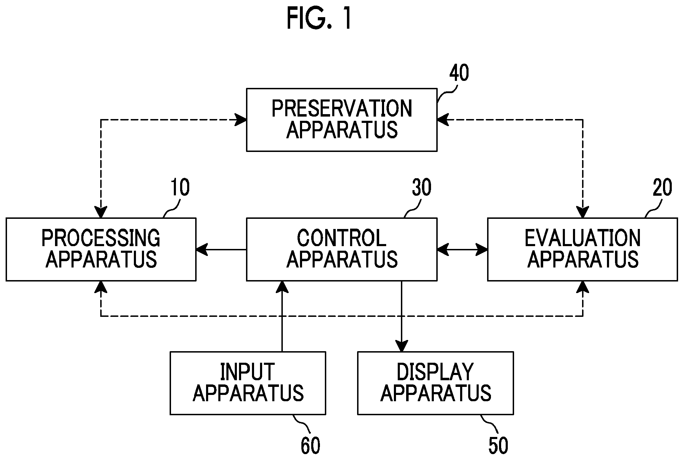

Hereinafter, with reference to the drawings, a detailed description will be made of a biological sample evaluation system according to an embodiment of the present invention. Fig. 1 is a block diagram illustrating a schematic configuration of a biological sample evaluation system of the present embodiment.

The biological sample evaluation system of the present embodiment comprises, as illustrated in Fig. 1, a processing apparatus 10, an evaluation apparatus 20, a control apparatus 30, a preservation apparatus 40, a display apparatus 50, and an input apparatus 60.

First, in a case where a biological sample is evaluated in the biological sample evaluation system of the present embodiment, a plurality of biological samples of an identical type are prepared. The plurality of biological samples of an identical type are a cell group regarded to be substantially identical in evaluation of the nature or the like of the cell group. For example, in a case where a biological sample is a cell, a plurality of biological samples are cultured from a single cell strain, and are created by dispensing the single cell strain to a plurality of containers of an identical type in an identical amount. In a case where even biological samples cultured from a different cell strain and are regarded to be substantially identical in evaluation of the nature or the like of a cell group, the biological samples may be used as a plurality of biological samples of an identical type.

Examples of biological samples include pluripotent stem cells such as induced pluripotent stem cells (iPS) and embryonic stem cells (ES), cells of a nerve, skin, cardiac muscle, or liver differentiated and induced from a stem cell, or cells of skin, a retina, cardiac muscle, a blood corpuscle, a nerve, or an organ extracted from a human body. As a container, for example, a petri dish may be used, a multi-well plate may be used, and different wells of a single multi-well plate may be used.

Creation of a plurality of biological samples may be manually performed by a user, and may be automatically performed by using, for example, an apparatus configured with a mechanism sucking cells, a robot arm, and the like.

The processing apparatus 10 performs an identical process on a plurality of biological samples of an identical type. A process performed by the processing apparatus 10 includes, for example, addition of a chemical of which a working mechanism is desired to be examined, or a physical stimulus such as irradiation with light. However, a process is not limited to such a process, and any process may be performed as long as the process is a necessary process for evaluating a biological sample. A process on each biological sample is performed by automatically adding a chemical to a container storing the biological sample by using an automatic dispensing device or the like.

The evaluation apparatus 20 evaluates the plurality of biological samples created as described above. Specifically, the evaluation apparatus 20 of the present embodiment captures an image of each biological sample in order to evaluate a morphological change of the biological sample, and comprises, for example, a phase difference microscope, a differential interference microscope, a bright-field microscope, or a fluorescence microscope. Such a microscope comprises an imaging element such as a complementary metal-oxide semiconductor (CMOS) sensor or a charge-coupled device (CCD) sensor.

Evaluation of a biological sample is not limited to capturing an image of the biological sample as in the present embodiment, and may be to measure the intensity of fluorescent light emitted from the biological sample, and may be to detect light emitted from the biological sample so as to perform spectral analysis of the light.

The preservation apparatus 40 comprises an incubator, and preserves a plurality of biological samples. The biological samples stored in the preservation apparatus 40 are extracted from the preservation apparatus 40, and are supplied to the processing apparatus 10 and the evaluation apparatus 20, in a case where a process and evaluation are performed. The biological samples are moved between the processing apparatus 10 and the preservation apparatus 40, between the evaluation apparatus 20 and the preservation apparatus 40, and between the processing apparatus 10 and the evaluation apparatus 20, as necessary. The biological samples may be automatically moved by using a robot arm, and may be automatically moved by using a conveyer belt or a turn table.

The display apparatus 50 comprises a display device such as a liquid crystal display. The display apparatus 50 displays a selection screen for receiving selection of each operation mode which will be described later and displays an input window for receiving various setting inputs from a user under the control of the control apparatus 30. An image of a biological sample captured by the evaluation apparatus 20 and other evaluation results may be displayed.

The input apparatus 60 comprises an input device such as a keyboard or a mouse, and receives various setting inputs from a user.

The control apparatus 30 is configured with a computer comprising a central processing unit (CPU), a memory, and the like, and controls the entire biological sample evaluation system. The control apparatus 30 is an apparatus in which a biological sample evaluation control program according to an embodiment of the present invention is installed in the computer, and a function of the control apparatus 30 is realized by the CPU executing the program. The biological sample evaluation control program is recorded on a recording medium such as a digital versatile disc (DVD) or a compact disc read only memory (CD-ROM) and is distributed, and is installed in a computer from the recording medium. The biological sample evaluation control program is stored in a storage device of a server computer connected to a network or a network storage in an accessible state from the outside, and is downloaded to and installed in a computer in response to a request.

The control apparatus 30 controls a time interval of each process on each biological sample in the processing apparatus 10 and a time interval of each evaluation of each biological sample in the evaluation apparatus 20. Specifically, even in a case where the shortest evaluation operation time of the evaluation apparatus 20 is longer than a desired time interval for observation, the control apparatus 30 of the present embodiment controls a time interval of each process on each biological sample and a time interval of each evaluation of each biological sample by using a plurality of biological samples of an identical type such that the biological sample can be evaluated at the desired time interval.

Hereinafter, a description will be made of control of the control apparatus 30 in a case where a biological sample is evaluated every x seconds from the time at which T seconds elapses after a process on the biological sample is performed. In addition, x seconds is the desired time interval. Herein, it is assumed that n biological samples are prepared.

First, the control apparatus 30 sets a time interval t1 of each process on each biological sample in the processing apparatus 10 and a time interval t2 of each evaluation of each biological sample in the evaluation apparatus 20. The time interval t1 of each process is a time interval which is equal to or more than the shortest process operation time of the processing apparatus 10, and is a time interval which is set and input by a user. The shortest process operation time is the shortest time required for the processing apparatus 10 to perform a process on a single biological sample. The time interval t2 of each evaluation is a time interval which is equal to or more than the shortest evaluation operation time and is obtained by adding the desired time interval x to the time interval t1 of each process. In other words, t2=t1+x. The time interval t2 of each evaluation is also a time interval set and input by the user.

The control apparatus 30 sets the time interval t1 of each process and the time interval t2 of each evaluation on the basis of setting inputs from the user, controls the processing apparatus 10 to sequentially perform n processes on the respective biological samples at the time interval t1 from the reference time of 0 seconds, and also controls the evaluation apparatus 20 to sequentially perform n evaluations of the respective biological samples at the time interval t2 from the time at which T seconds elapses from the reference time of 0 seconds. The elapse time T may be zero seconds.

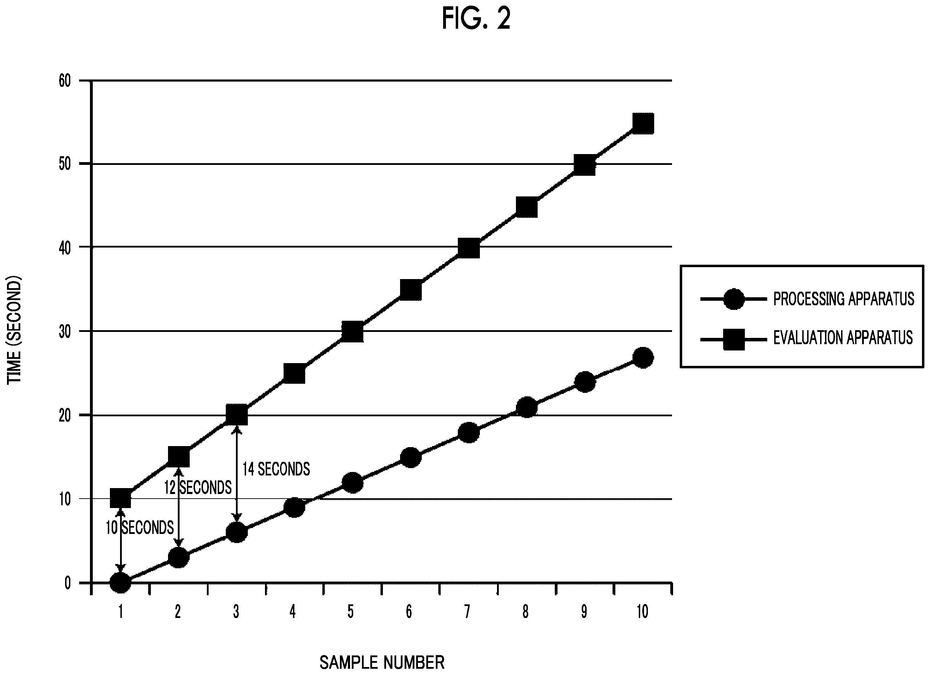

Fig. 2 is a graph illustrating process timings (indicated by black circles) and evaluation timings (indicated by black squares) for respective ten biological samples in a case where the ten biological samples are prepared, the time interval t1 of each process on each biological sample is set to 3 seconds, the time interval t2 of each evaluation of each biological sample is set to 5 seconds, and the elapse time T from the reference time is set to 10 seconds. Herein, the time interval t1 of each process is referred to as the shortest process operation time, and the time interval t2 of each evaluation is referred to as the shortest evaluation operation time.

According to each process timing and each evaluation timing illustrated in Fig. 2, for example, with respect to the biological sample with the sample number 1, an evaluation is performed at the time at which 10 seconds elapses after a process is performed; with respect to the biological sample with the sample number 2, an evaluation is performed at the time at which 12 seconds elapses after a process is performed; and, with respect to the biological sample with the sample number 3, an evaluation is performed at the time at which 14 seconds elapses after a process is performed. Therefore, an evaluation is substantially performed at a time interval of 2 seconds, that is, the evaluation can be performed at a time interval shorter than the shortest evaluation operation time.

In the above description, the time interval t2 of each evaluation is an interval obtained by adding the desired time interval x to the time interval t1 of each process, but is not limited thereto, and may be an interval obtained by subtracting the desired time interval x from the time interval t1 of each process. In other words, t2=tl-x. However, as described above, the time interval t2 is a time which is equal to or more than the shortest evaluation operation time.

In this case, the control apparatus 30 controls the processing apparatus 10 to sequentially perform n processes on the respective biological samples at the time interval t1 from the reference time of 0 seconds, and also controls the evaluation apparatus 20 to sequentially perform n evaluations of the respective biological samples at the time interval t2 from the time point of {T+(n-1)x} seconds.

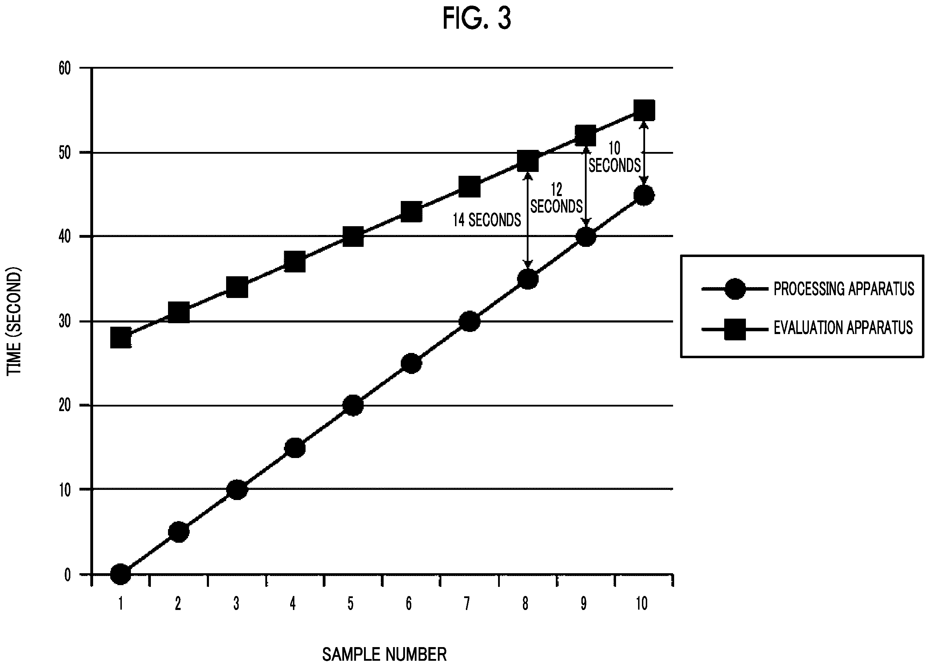

Fig. 3 is a graph illustrating process timings (indicated by black circles) and evaluation timings (indicated by black squares) for respective ten biological samples in a case where the ten biological samples are prepared, the time interval t1 of each process on each biological sample is set to 5 seconds, the time interval t2 of each evaluation of each biological sample is set to 3 seconds, and the elapse time T from the reference time is set to 28 seconds. Herein, the time interval t1 of each process is referred to as the shortest process operation time, and the time interval t2 of each evaluation is referred to as the shortest evaluation operation time.

According to each process timing and each evaluation timing illustrated in Fig. 3, for example, with respect to the biological sample with the sample number 10, an evaluation is performed at the time at which 10 seconds elapses after a process is performed; with respect to the biological sample with the sample number 9, an evaluation is performed at the time at which 12 seconds elapses after a process is performed; and, with respect to the biological sample with the sample number 8, an evaluation is performed at the time at which 14 seconds elapses after a process is performed. Therefore, an evaluation is substantially performed at an interval of 2 seconds, that is, the evaluation can be performed at an interval shorter than the shortest evaluation operation time.

Here, in the examples illustrated in Figs. 2 and 3, the time interval t1 of each process is set as the shortest process operation time, and the time interval t2 of each evaluation is set as the shortest evaluation operation time, but this is only an example, and, as described above, an evaluation can be performed at the desired time interval x in a case where the time interval t1 of each process and the time interval t2 of each evaluation are respectively equal to or more than the shortest process operation time and the shortest evaluation operation time, and satisfy a relationship of t2=t1+x or t2=t1-x.

Here, in a case where an evaluation result is desired to be obtained as soon as possible, or a constraint time of a human monitoring an evaluation is shortened, the time interval t1 of each process and the time interval t2 of each evaluation are preferably set such that the time required to complete all processes and evaluations for the respective biological samples is shortest.

In a case where the stability of the processing apparatus 10, the evaluation apparatus 20, or the entire biological sample evaluation system is checked, the stability of an operation is preferably checked by operating the biological sample evaluation system for a long period of time to some extent. In other words, the time interval t1 of each process and the time interval t2 of each evaluation are preferably set such that the time required to complete all processes and evaluations for the respective biological samples is any time designated by the user.

Therefore, the control apparatus 30 of the present embodiment has a basic operation mode for controlling the processing apparatus 10 and the evaluation apparatus 20 on the basis of the time interval t1 of each process and the time interval t2 of each evaluation which are set and input by the user, and also has various operation modes corresponding to the user's request. An operation mode selection screen is displayed on, for example, the display apparatus 50 in order to select various operation modes including the basic operation mode. The user selects any operation mode on the selection screen by using the input apparatus 60, and thus the control apparatus 30 sets the time interval t1 of each process and the time interval t2 of each evaluation for each biological sample corresponding to the operation mode.

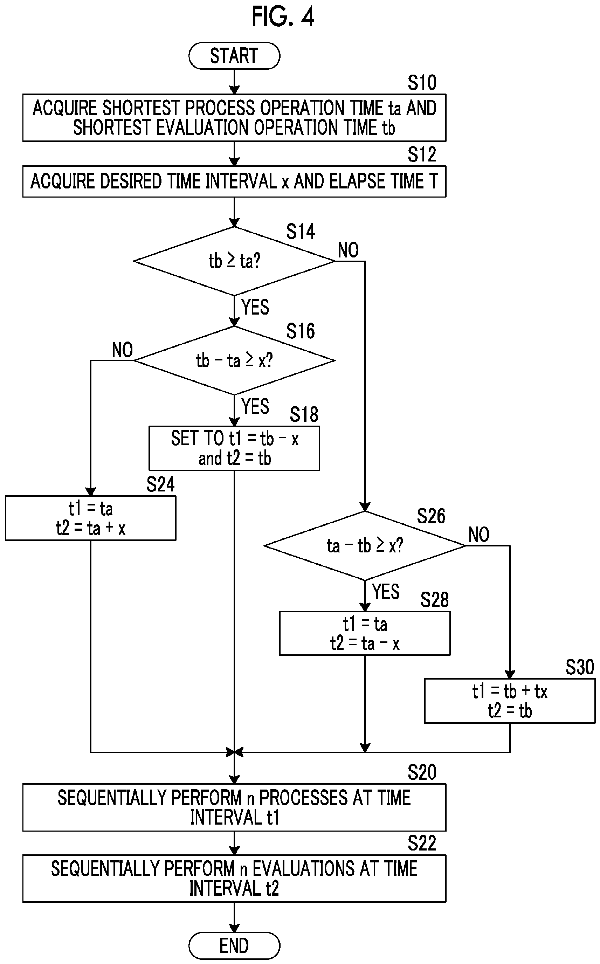

Hereinafter, a description will be made of a method of setting the time interval t1 of each process and the time interval t2 of each evaluation for each biological sample in each operation mode. First, with reference to a flowchart in Fig. 4, a description will be made of a shortest-time operation mode of setting the time interval t1 of each process and the time interval t2 of each evaluation such that the time required to complete all processes and evaluations for the respective biological samples is shortest.

In a case where the shortest-time operation mode is selected, the control apparatus 30 acquires a shortest process operation time ta and a shortest evaluation operation time tb (S10). The shortest process operation time ta and the shortest evaluation operation time tb may be set and input by the user by using the input apparatus 60, and may be stored in and acquired from a memory or the like of the processing apparatus 10, the evaluation apparatus 20, or the control apparatus 30. In a case where the user sets and inputs the times, an input window may be displayed on the display apparatus 50.

Next, the control apparatus 30 acquires the desired evaluation time interval x and the elapse time T from the reference time (S12). The desired evaluation time interval x and the elapse time T from the reference time may be set and input by the user by using the input apparatus 60, and may be stored in and acquired from the memory or the like of the control apparatus 30. In a case where the user sets and inputs the times, an input window may be displayed on the display apparatus 50.

The control apparatus 30 sets the time interval t1 of each process and the time interval t2 of each evaluation for each biological sample as follows on the basis of the shortest process operation time ta, the shortest evaluation operation time tb, the desired time interval x, and the elapse time T from the reference time.

In a case where tb≥ta and tb-ta≥x (YES in S14 and YES in S16), the control apparatus 30 sets the time interval t1 of each process on each biological sample to (tb-x), and sets the time interval t2 of each evaluation of each biological sample to tb (S18).

The control apparatus 30 controls the processing apparatus 10 to sequentially perform n processes on the respective biological samples at the time interval t1 (=(tb-x)) from the time point of 0 seconds (S20), and also controls the evaluation apparatus 20 to sequentially perform n evaluations of the respective biological samples at the time interval t2 (=tb) from the time at which T seconds elapses from the time point of 0 seconds (S22).

In a case where tb≥ta and tb-ta<x (YES in S14 and NO in S16), the control apparatus 30 sets the time interval t1 of each process on each biological sample to ta, and sets the time interval t2 of each evaluation of each biological sample to (ta+x) (S24).

The control apparatus 30 controls the processing apparatus 10 to sequentially perform n processes on the respective biological samples at the time interval t1 (=(ta)) from the time point of 0 seconds (S20), and also controls the evaluation apparatus 20 to sequentially perform n evaluations of the respective biological samples at the time interval t2 (=ta+x) from the time at which T seconds elapses from the time point of 0 seconds (S22).

In a case where tb<ta and ta-tb≥x (NO in S14 and YES in S26), the control apparatus 30 sets the time interval t1 of each process on each biological sample to ta, and sets the time interval t2 of each evaluation of each biological sample to (ta-x) (S28).

The control apparatus 30 controls the processing apparatus 10 to sequentially perform n processes on the respective biological samples at the time interval t1 (=(ta)) from the time point of 0 seconds (S20), and also controls the evaluation apparatus 20 to sequentially perform n evaluations of the respective biological samples at the time interval t2 (=ta-x) from the time at which {T+(n-1)x} seconds elapses from the time point of 0 seconds (S22).

In a case where tb<ta and ta-tb<x (NO in S14 and NO in S26), the control apparatus 30 sets the time interval t1 of each process on each biological sample to (tb+x), and sets the time interval t2 of each evaluation of each biological sample to tb (S30).

The control apparatus 30 controls the processing apparatus 10 to sequentially perform n processes on the respective biological samples at the time interval t1 (=tb+x) from the time point of 0 seconds (S20), and also controls the evaluation apparatus 20 to sequentially perform n evaluations of the respective biological samples at the time interval t2 (=tb) from the time at which {T+(n-1)x} seconds elapses from the time point of 0 seconds (S22).

In a case where the shortest-time operation mode is selected, the control apparatus 30 sets the time interval t1 of each process and the time interval t2 of each evaluation for each biological sample in the above-described way, and can thus make the time required to complete all processes and evaluations for the respective biological samples shortest at all times.

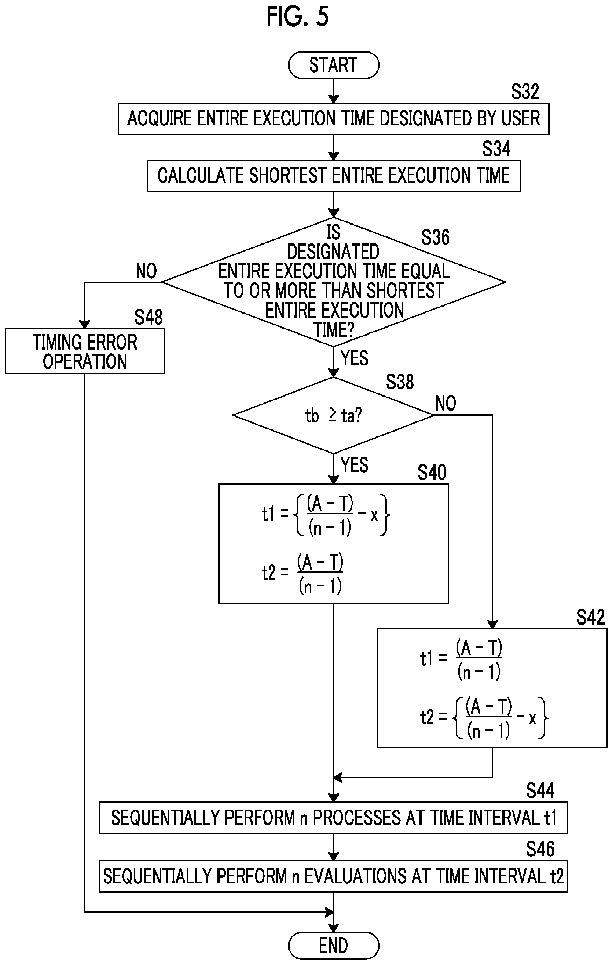

Next, with reference to a flowchart in Fig. 5, a description will be made of an any-time operation mode of setting the time interval t1 of each process and the time interval t2 of each evaluation such that the time required to complete all processes and evaluations for the respective biological samples is any time designated by the user.

In a case where the any-time operation mode is selected, the control apparatus 30 acquires any entire execution time A which is set and input by the user by using the input apparatus 60 (S32). The entire execution time A is the time required to complete all processes and evaluations for the respective biological samples. Also with respect to input of the entire execution time A, an input window may be displayed on the display apparatus 50.

Next, the control apparatus 30 calculates the shortest entire execution time of the biological sample evaluation system (S34). The shortest entire execution time is calculated by using the same method as that in the above-described shortest-time operation mode. In a case where tb≥ta and tb-ta≥x, the shortest entire execution time is {T+(n-1)tb} seconds, and, on the other hand, in a case where tb≥ta and tb-ta<x, the shortest entire execution time is {T+(n-1)(ta+x)} seconds. In a case where tb<ta and ta-tb≥x, the shortest entire execution time is {T+(n-1)ta} seconds, and, on the other hand, in a case where tb<ta and ta-tb<x, the shortest entire execution time is {T+(n-1)(tb+x)} seconds.

In a case where the entire execution time A designated by the user is equal to or more than the shortest entire execution time (YES in S36), the control apparatus 30 sets the time interval t1 of each process and the time interval t2 of each evaluation for each biological sample as follows.

In a case where tb≥ta (YES in S38), the control apparatus 30 sets the time interval t1 of each process on each biological sample to {(A-T)/(n-1)-x}, and sets the time interval t2 of each evaluation of each biological sample to (A-T)/(n-1) (S40).

The control apparatus 30 controls the processing apparatus 10 to sequentially perform n processes on the respective biological samples at the time interval t1 from the time point of 0 seconds (S44), and also controls the evaluation apparatus 20 to sequentially perform n evaluations of the respective biological samples at the time interval t2 from the time at which T seconds elapses from the time point of 0 seconds (S46).

In a case where tb<ta (NO in S38), the control apparatus 30 sets the time interval t1 of each process on each biological sample to (A-T)/(n-1), and sets the time interval t2 of each evaluation of each biological sample to {(A-T)/(n-1)-x} (S42).

The control apparatus 30 controls the processing apparatus 10 to sequentially perform n processes on the respective biological samples at the time interval t1 from the time point of 0 seconds (S44), and also controls the evaluation apparatus 20 to sequentially perform n evaluations of the respective biological samples at the time interval t2 from the time at which T seconds elapses from the time point of 0 seconds (S46).

On the other hand, in a case where the entire execution time A designated by the user is shorter than the shortest entire execution time, that is, the time interval t1 of each process and the time interval t2 of each evaluation satisfying the condition of the entire execution time A cannot be determined, the control apparatus 30 performs a timing error operation (S48).

As the timing error operation, the control apparatus 30 displays an error message or the like on the display apparatus 50 so as to perform a warning operation. The warning operation is not limited to display of an error message, and a warning sound may be issued, or an icon indicating an error may be displayed.

As the timing error operation, the time interval t1 of each process and the time interval t2 of each evaluation which are realizable may be displayed on the display apparatus 50. Specifically, for example, the time interval t1 of each process and the time interval t2 of each evaluation in the shortest-time operation mode may be displayed on the display apparatus 50, and a selection of whether or not the shortest-time operation mode is performed may be received.

According to the any-time operation mode, the biological sample evaluation system can be operated for any execution time designated by the user, and thus the stability of the operation of the system can be checked. In a case where the condition of the any-time operation mode cannot be satisfied, the user can perform appropriate adjustment by performing the timing error operation.

Next, a description will be made of an execution time restriction operation mode in which the time interval t1 of each process and the time interval t2 of each evaluation are set such that the time required for a process and an evaluation for a single biological sample is equal to or less than a preset time.

A biological sample such as a cell is preserved in the preservation apparatus 40 in which humidity, a temperature, and a carbon dioxide concentration are appropriately managed, but is temporarily extracted from the preservation apparatus 40 in a case where a process in the processing apparatus 10 and an evaluation in the evaluation apparatus 20 are performed. An environment outside the preservation apparatus 40 is often in the air and is different from an environment inside the preservation apparatus 40. In other words, a biological sample is exposed to an environment in which the biological sample hardly survives during a process in the processing apparatus 10 and an evaluation in the evaluation apparatus 20. In a case where a period of time in which a biological sample is exposed to the air is long, survival of the biological sample is influenced, and thus it is preferable to restrict the time required for a process and an evaluation for a single biological sample. The execution time restriction operation mode is an operation mode which is set in consideration of the above-described circumstances.

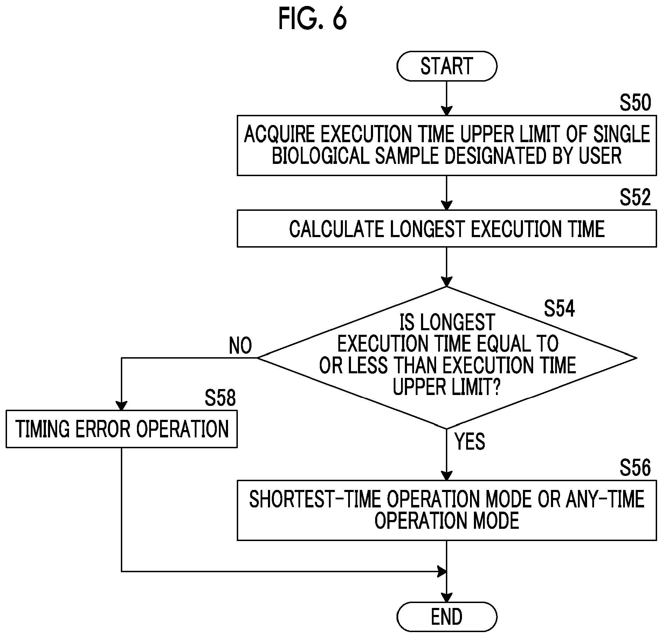

Hereinafter, the execution time restriction operation mode will be described with reference to a flowchart in Fig. 6.

In a case where the execution time restriction operation mode is selected, the control apparatus 30 acquires an execution time upper limit B which is set and input by the user by using the input apparatus 60 (S50).

Next, the control apparatus 30 calculates the longest execution time for a single biological sample (S52). The longest execution time is the longest execution time among execution times required for a process and an evaluation for each biological sample, and is {T+(n-1)x} seconds in a case where the elapse time T from the reference time is indicated by T, the number of biological samples is indicated by n, and the desired evaluation time interval is indicated by x.

In a case where the longest execution time is equal to or less than the execution time upper limit B designated by the user (YES in S54), the control apparatus 30 performs the shortest-time operation mode or the any-time operation mode. An operation mode to be performed of the shortest-time operation mode and the any-time operation mode may be set in advance, and may be selected on a selection screen by the user.

On the other hand, in a case where the longest execution time exceeds the execution time upper limit B designated by the user (NO in S54), that is, the time interval t1 of each process and the time interval t2 of each evaluation satisfying the condition of the execution time upper limit B cannot be determined, the control apparatus 30 performs a timing error operation (S58).

As the timing error operation, the control apparatus 30 displays an error message or the like on the display apparatus 50 so as to perform a warning operation. The warning operation is not limited to display of an error message, and a warning sound may be issued, or an icon indicating an error may be displayed.

As the timing error operation, a realizable execution time for a single biological sample may be displayed on the display apparatus 50. Specifically, for example, the above {T+(n-1)x} may be displayed on the display apparatus 50, and such a condition may be used without being changed, or a change of the elapse time T from the reference time, the number n of biological samples, or the desired evaluation time interval x may be received.

According to the execution time operation mode, it is possible to automatically determine whether or not the time required for a process and an evaluation for a single biological sample will be able to be equal to or less than a preset time, and, in a case where realization thereof is not possible, a user can perform appropriate adjustment.

Next, a description will be made of an apparatus use time restriction operation mode in which the time interval t1 of each process and the time interval t2 of each evaluation are set such that a total use time of the processing apparatus 10 or the evaluation apparatus 20 is equal to or less than a preset time.

There is a case where other users use the processing apparatus 10 or the evaluation apparatus 20 in other evaluations in addition to a single user. In this case, a process and an evaluation are preferably performed by restricting a total use time of the processing apparatus 10 or the evaluation apparatus 20. The apparatus use time restriction operation mode is an operation mode which is set in consideration of the above-described circumstances.

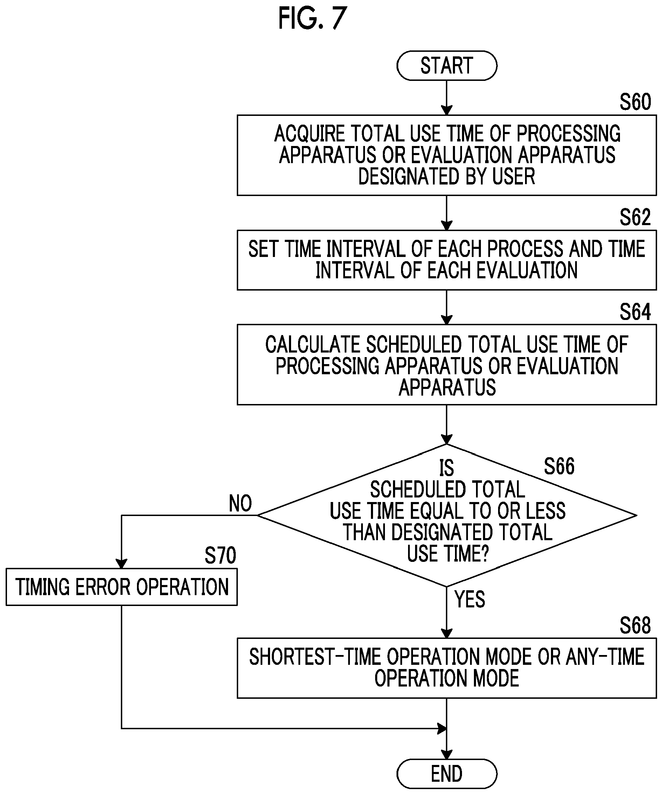

Hereinafter, the apparatus use time restriction operation mode will be described with reference to a flowchart in Fig. 7.

In a case where the apparatus use time restriction operation mode is selected, the control apparatus 30 acquires a total use time of the processing apparatus 10 or the evaluation apparatus 20 which is set and input by the user by using the input apparatus 60 (S60).

Next, the control apparatus 30 receives a selection of any one of the shortest-time operation mode and the any-time operation mode, and sets the time interval t1 of each process and the time interval t2 of each evaluation according to the selected operation mode (S62).

Next, the control apparatus 30 calculates a scheduled total use time of the processing apparatus 10 or the evaluation apparatus 20 on the basis of the set time interval t1 of each process or the set time interval t2 of each evaluation (S64). The scheduled total use time of the processing apparatus 10 is calculated as (n-1)t1, and the scheduled total use time of the evaluation apparatus 20 is calculated as (n-1)t2.

In a case where the scheduled total use time of the processing apparatus 10 or the evaluation apparatus 20 is equal to or less than the total use time of the processing apparatus 10 or the evaluation apparatus 20 designated by the user (YES in S66), the control apparatus 30 performs the shortest-time operation mode or the any-time operation mode selected by the user.

On the other hand, in a case where the scheduled total use time of the processing apparatus 10 or the evaluation apparatus 20 exceeds the total use time of the processing apparatus 10 or the evaluation apparatus 20 designated by the user (NO in S66), that is, the time interval t1 of each process and the time interval t2 of each evaluation satisfying the condition of the total use time designated by the user cannot be determined, the control apparatus 30 performs a timing error operation (S70).

As the timing error operation, the control apparatus 30 displays an error message or the like on the display apparatus 50 so as to perform a warning operation. The warning operation is not limited to display of an error message, and a warning sound may be issued, or an icon indicating an error may be displayed.

As the timing error operation, a realizable total use time of the processing apparatus 10 or the evaluation apparatus 20 may be displayed on the display apparatus 50. Specifically, for example, the above (n-1)t1 or (n-1)t2 may be displayed on the display apparatus 50, and such a condition may be used without being changed, or a change of the number n of biological samples or the desired evaluation time interval x may be received.

According to the apparatus use time restriction operation mode, it is possible to automatically determine whether or not a total use time of the processing apparatus 10 or the evaluation apparatus 20 will be able to be equal to or less than a preset time, and, in a case where realization thereof is not possible, a user can perform appropriate adjustment. Consequently, it is possible to improve the usage efficiency of the processing apparatus 10 or the evaluation apparatus 20.

In the biological sample evaluation system of the embodiment, in a case where the processing apparatus 10 or the evaluation apparatus 20 wrongly operates or fails and thus causes an error, a preset apparatus error operation may be performed.

As the apparatus error operation, the control apparatus 30 displays an apparatus error message or the like on the display apparatus 50 so as to perform a warning operation. The warning operation is not limited to display of an apparatus error message, and a warning sound may be issued, or an icon indicating an apparatus error may be displayed.

Alternatively, as the apparatus error operation, the control apparatus 30 may stop the processing apparatus 10 or the evaluation apparatus 20. However, in a case where an apparatus error occurs in either the processing apparatus 10 or the evaluation apparatus 20, the one apparatus may be stopped, and the other apparatus may be continuously operated.

For example, in a case where an apparatus error occurs in only the evaluation apparatus 20, the evaluation apparatus 20 may be stopped, only a process may be continuously performed by the processing apparatus 10, and an evaluation may be performed by the user through visual observation. Alternatively, for example, in a case where an apparatus error occurs in only the processing apparatus 10, the processing apparatus 10 may be stopped, only an evaluation may be continuously performed by the evaluation apparatus 20, and only an evaluation of a biological sample having undergone an appropriate process may be referred to.

### Explanation of References

- 10:: processing apparatus
- 20:: evaluation apparatus
- 30:: control apparatus
- 40:: preservation apparatus
- 50:: display apparatus
- 60:: input apparatus

## Claims

1. A biological sample evaluation system comprising a processing apparatus (10), an evaluation apparatus (20) and a control apparatus (30) that controls the processing apparatus (10) and the evaluation apparatus (20),
**characterized in that**:
the processing apparatus (10) performs an identical process on a plurality of biological samples of an identical type;
the evaluation apparatus (20) evaluates the plurality of biological samples; and
the control apparatus (30) that controls a time interval (t1) of each process on each of the biological samples in the processing apparatus (10) and a time interval (t2) of each evaluation of each biological sample in the evaluation apparatus (20),
wherein the control apparatus (30) sets the time interval (t1) of each process to a time interval which is equal to or more than a shortest process operation time (ta) of the processing apparatus (10), sets the time interval (t2) of each evaluation to a time interval which is equal to or more than a shortest evaluation operation time (tb) of the evaluation apparatus (20) and is obtained by adding or subtracting a desired evaluation time interval shorter than the shortest evaluation operation time (tb) to or from the time interval (t1) of each process,
the processing apparatus (10) sequentially performs (S20) processes on the respective biological samples with the time interval (t1) between each process from a reference time, and
the evaluation apparatus (20) sequentially evaluates (S22) the respective biological samples with the time interval between each evaluation of each biological sample from a time point at which a preset time elapses from the reference time to obtain a plurality of evaluation results at the desired time interval.

2. The biological sample evaluation system according to claim 1, wherein
in a case where the shortest process operation time (ta) is equal or smaller than the shortest evaluation operation time (tb) (S14:YES) and the desired evaluation time interval (x) is equal or smaller than the value of the shortest evaluation operation time (tb) minus the shortest process operation time (ta) (S26:YES), the control apparatus (30) sets the time interval (t1) of each process to the value of the shortest evaluation operation time (tb) minus the desired evaluation time interval (x), and sets the time interval (t2) of each evaluation of each biological sample to the shortest evaluation operation time (tb) (S18),
in a case where the shortest process operation time (ta) is larger than the shortest evaluation operation time (tb) (S14:NO) and the desired evaluation time interval (x) is equal or smaller than the value of the shortest process operation time (ta) minus the shortest evaluation operation time (tb) (S26:YES), the control apparatus (30) sets the time interval (t1) of each process to the value of the shortest process operation time (ta), and sets the time interval (t2) of each evaluation of each biological sample to the value of the shortest process operation time (ta) minus the desired evaluation time interval (x) (S28).

3. The biological sample evaluation system according to claim 1,
wherein the control apparatus (30) acquires (S10) the shortest process operation time (ta) and the shortest evaluation operation time (tb), and sets the time interval (t1) of each process and the time interval (t2) of each evaluation on the basis of the acquired shortest process operation time (ta) and shortest evaluation operation time (tb), the desired time interval (x) and the elapsed time T from the reference time.

4. The biological sample evaluation system according to claim 3,
wherein the control apparatus (30) has an operation mode in which the time interval (t1) of each process and the time interval (t2) of each evaluation are set such that a time required to complete all processes and evaluations for the plurality of biological samples is shortest.

5. The biological sample evaluation system according to claim 3 or 4,
wherein the control apparatus (30) has an operation mode in which the time interval (t1) of each process and the time interval (t2) of each evaluation are set such that a time required to complete all processes and evaluations for the plurality of biological samples is any time designated in advance.

6. The biological sample evaluation system according to any one of claims 1 to 5,
wherein the control apparatus (30) has an operation mode in which the time interval (t1) of each process and the time interval (t2) of each evaluation are set such that a time required for the process and the evaluation for a single biological sample is equal to or less than a preset time.

7. The biological sample evaluation system according to any one of claims 1 to 6,
wherein the control apparatus (30) has an operation mode in which the time interval (t1) of each process and the time interval (t2) of each evaluation are set such that a total use time of the processing apparatus (10) is equal to or less than a preset time.

8. The biological sample evaluation system according to any one of claims 1 to 7,
wherein the control apparatus (30) has an operation mode in which the time interval (t1) of each process and the time interval (t2) of each evaluation are set such that a total use time of the evaluation apparatus (20) is equal to or less than a preset time.

9. The biological sample evaluation system according to any one of claims 5 to 8,
wherein, in a case where the control apparatus (30) is not able to determine the time interval (t1) of each process and the time interval (t2) of each evaluation satisfying a condition of the operation mode, the control apparatus (30) performs a preset timing error operation.

10. The biological sample evaluation system according to claim 9,
wherein the control apparatus (30) performs a warning operation as the timing error operation.

11. The biological sample evaluation system according to claim 9 or 10,
wherein, as the timing error operation, the control apparatus (30) displays the time interval (t1) of each process and the time interval (t2) of each evaluation which are realizable on a display apparatus (50).

12. The biological sample evaluation system according to any one of claims 1 to 11,
wherein, in a case where the processing apparatus (10) or the evaluation apparatus (20) causes an error, the control apparatus (30) performs a preset apparatus error operation wherein preferably the control apparatus (30) performs a warning operation as the apparatus error operation.

13. The biological sample evaluation system according to claim 12,
wherein the control apparatus (30) stops the processing apparatus (10) and the evaluation apparatus (20) as the apparatus error operation.

14. The biological sample evaluation system according to claim 11 or 12,
wherein, in a case where either the processing apparatus (10) or the evaluation apparatus (20) causes an error, the control apparatus (30) stops one apparatus, and continuously operates the other apparatus.

15. A biological sample evaluation method comprising:
causing a processing apparatus (10) to sequentially perform processes on a plurality of biological samples of an identical type from a reference time;
causing an evaluation apparatus (20) to sequentially evaluate the plurality of biological samples from a time point at which a preset time elapses from the reference time to obtain a plurality of evaluation results at a desired evaluation time interval (x), wherein a time interval (t1) of each process on each of the biological samples is set to a time interval which is equal to or more than a shortest process operation time (ta) of the processing apparatus (10), and a time interval (t2) of each evaluation of each of the biological samples is set to a time interval which is equal to or more than a shortest evaluation operation time (tb) of the evaluation apparatus (20) and is obtained by adding or subtracting the desired evaluation time interval (x) to or from the time interval (t1) of each process, the desired evaluation time interval (x) being shorter than the shortest evaluation operation time (tb),
sequentially performing processes on the respective biological samples at the time interval of each process from a reference time (t1), and
sequentially evaluating the respective biological samples at the term interval of each evaluation from a time point at which a preset time elapses from the reference time to obtain a plurality of evaluation results at the desired evaluation time interval.

16. A non-transitory computer readable recording medium storing a biological sample evaluation control program for realizing a function of a control apparatus (30) in a biological sample evaluation system comprising a processing apparatus (10) that performs an identical process on a plurality of biological samples of an identical type, an evaluation apparatus (20) that evaluates the plurality of biological samples, and the control apparatus (30) that controls a time interval (t1) of each process on each of the biological samples in the processing apparatus (10) and a time interval (t2) of each evaluation of each biological sample in the evaluation apparatus (20), the program causing the control apparatus (30) to execute:
a procedure of setting the time interval (t1) of each process to a time interval which is equal to or more than a shortest process operation time (ta) of the processing apparatus (10);
a procedure of setting the time interval (t2) of each evaluation to a time interval which is equal to or more than a shortest evaluation operation time (tb) of the evaluation apparatus (20) and is obtained by adding or subtracting a desired evaluation time interval (x), which is shorter than the shortest evaluation operation time (tb) to or from the time interval (t1) of each process;
a procedure of sequentially performing processes on the respective biological samples at the time interval (t1) of each process from a reference time; and
a procedure of sequentially evaluating the respective biological samples at the time interval (t2) of each evaluation from a time point at which a preset time elapses from the reference time to obtain a plurality of evaluation results at the desired evaluation time interval (x).

## Patentansprüche

1. Bewertungssystem für biologische Proben, umfassend eine Verarbeitungsvorrichtung (10), eine Bewertungsvorrichtung (20) und eine Steuervorrichtung (30), welche die Verarbeitungsvorrichtung (10) und die Bewertungsvorrichtung (20) steuert,
**dadurch gekennzeichnet, dass**
die Verarbeitungsvorrichtung (10) einen identischen Prozess an einer Mehrzahl biologischer Proben identischen Typs ausführt;
die Bewertungsvorrichtung (20) die mehreren biologischen Proben bewertet; und die Steuervorrichtung (30), ein Zeitintervall (t1) jedes Prozesses an jeder der biologischen Proben in der Verarbeitungsvorrichtung (10) und ein Zeitintervall (t2) jeder Bewertung jeder biologischen Probe in der Bewertungsvorrichtung (20) steuert,
wobei die Steuervorrichtung (30) das Zeitintervall (t1) jedes Prozesses auf ein Zeitintervall einstellt, welches gleich oder größer ist als eine kürzeste Prozessbetriebszeit (ta) der Verarbeitungsvorrichtung (10), das Zeitintervall (t2) jeder Bewertung auf ein Zeitintervall einstellt, welches gleich oder größer ist als eine kürzeste Bewertungsbetriebszeit (tb) der Bewertungsvorrichtung (20), und gewonnen wird durch Addieren oder Subtrahieren eines gewünschten Bewertungszeitintervalls kürzer als die kürzeste Bewertungsbetriebszeit (tb) auf oder von dem Zeitintervall (t1) jedes Prozesses,
die Verarbeitungsvorrichtung (10) sequentiell Prozesse an jeweiligen biologischen Proben mit dem Zeitintervall (t1) zwischen jedem Prozess von einer Referenzzeit ausführt (S20), und die Bewertungsvorrichtung (20) sequentiell die jeweiligen biologischen Proben mit dem Zeitintervall zwischen jeder Bewertung jeder biologischen Probe ab einem Zeitpunkt bewertet (S22), zu dem eine voreingestellte Zeit ausgehend von der Referenzzeit verstreicht, um mehrere Bewertungsergebnisse zu dem gewünschten Zeitintervall zu gewinnen.

2. Bewertungssystem für biologische Proben nach Anspruch 1, bei dem
für den Fall, dass die kürzeste Prozessbetriebszeit (ta) gleich oder kleiner ist als die kürzeste Bewertungsbetriebszeit (tb) (S14: YES) und das gewünschte Bewertungszeitintervall (x) gleich oder kleiner ist als der Wert der kürzesten Bewertungsbetriebszeit (tb) minus die kürzeste Prozessbetriebszeit (ta) (S26: YES), die Steuervorrichtung (30) das Zeitintervall (t1) jedes Prozesses auf den Wert der kürzesten Bewertungsbetriebszeit (tb) minus das gewünschte Bewertungszeitintervall (x) einstellt, und das Zeitintervall (t2) zu jeder Bewertung jeder biologischen Probe auf die kürzeste Bewertungsbetriebszeit (tb) einstellt (S18),
für den Fall, dass die kürzeste Prozessbetriebszeit (ta) größer ist als die kürzeste Bewertungsbetriebszeit (tb) (S14: NO) und das gewünschte Bewertungszeitintervall (x) gleich oder kleiner ist als der Wert der kürzesten Prozessbetriebszeit (ta) minus die kürzeste Bewertungsbetriebszeit (tb) (S26: YES), die Steuervorrichtung (30) das Zeitintervall (t1) jedes Prozesses auf den Wert der kürzesten Prozessbetriebszeit (ta) einstellt und das Zeitintervall (t2) jeder Bewertung jeder biologischen Probe auf den Wert der kürzesten Prozessbetriebszeit (ta) minus das gewünschte Bewertungszeitintervall (x) einstellt (S28).

3. Bewertungssystem für biologische Proben nach Anspruch 1,
bei dem die Steuervorrichtung (30) die kürzeste Prozessbetriebszeit (ta) und die kürzeste Bewertungsbetriebszeit (tb) erfasst (S10) und das Zeitintervall (t1) jedes Prozesses und das Zeitintervall (t2) jeder Bewertung auf der Grundlage der kürzesten Prozessbetriebszeit (ta) und der kürzesten Bewertungsbetriebszeit (tb), des gewünschten Zeitintervalls (x) und der seit der Referenzzeit verstrichenen Zeit T einstellt.

4. Bewertungssystem für biologische Proben nach Anspruch 3,
bei dem die Steuervorrichtung (30) eine Betriebsart aufweist, in welcher das Zeitintervall (t1) jedes Prozesses und das Zeitintervall (t2) jeder Bewertung derart eingestellt werden, dass eine Zeit, die erforderlich ist zum Abschließen sämtlicher Prozesse und Bewertungen für die mehreren biologischen Proben, am kürzesten ist.

5. Bewertungssystem für biologische Proben nach Anspruch 3 oder 4,
bei dem die Steuervorrichtung (30) eine Betriebsart aufweist, in welcher das Zeitintervall (t1) für jeden Prozess und das Zeitintervall (t2) für jede Bewertung derart eingestellt werden, dass eine Zeit, die erforderlich ist zum Beenden sämtlicher Prozesse und Bewertungen für die mehreren biologischen Proben, jeder vorab festgelegten Zeit entspricht.

6. Bewertungssystem für biologische Proben nach einem der Ansprüche 1 bis 5,
bei dem die Steuervorrichtung (30) eine Betriebsart aufweist, in welcher das Zeitintervall (t1) jedes Prozesses und das Zeitintervall (t2) jeder Bewertung derart eingestellt werden, dass eine für den Prozess und die Bewertung einer einzelnen biologischen Probe erforderliche Zeit gleich oder kleiner ist als eine voreingestellte Zeit.

7. Bewertungssystem für biologische Proben nach einem der Ansprüche 1 bis 6,
bei dem die Steuervorrichtung (30) eine Betriebsart aufweist, in welcher das Zeitintervall (t1) jedes Prozesses und das Zeitintervall (t2) jeder Bewertung derart eingestellt werden, dass eine Gesamtnutzungszeit der Verarbeitungsvorrichtung (10) gleich oder kleiner als eine vorbestimmte Zeit ist.

8. Bewertungssystem für biologische Proben nach einem der Ansprüche 1 bis 7,
bei dem die Steuervorrichtung (30) eine Betriebsart aufweist, in welcher das Zeitintervall (t1) jedes Prozesses und das Zeitintervall (t2) jeder Bewertung derart eingestellt sind, dass eine Gesamtnutzungszeit der Bewertungsvorrichtung (20) gleich oder kleiner ist als eine voreingestellte Zeit.

9. Bewertungssystem für biologische Proben nach einem der Ansprüche 5 bis 8,
bei dem für den Fall, dass die Steuervorrichtung (30) nicht imstande ist, das Zeitintervall (t1) jedes Prozesses und das Zeitintervall (t2) jeder Bewertung zu bestimmen, welche eine Bedingung für die Betriebsart erfüllt, die Steuervorrichtung (30) eine voreingestellte Zeitfehleroperation ausführt.

10. Bewertungssystem für biologische Proben nach Anspruch 9,
bei dem die Steuervorrichtung (30) als Zeitfehleroperation einen Warnvorgang ausführt.

11. Bewertungssystem für biologische Proben nach Anspruch 9 oder 10,
bei dem als die Zeitfehleroperation die Steuervorrichtung das Zeitintervall (t1) jedes Prozesses und das Zeitintervall (t2) jeder Bewertung anzeigt, die auf einer Anzeigevorrichtung (50) realisierbar sind.

12. Bewertungssystem für biologische Proben nach einem der Ansprüche 1 bis 11,
bei dem für den Fall, dass die Verarbeitungsvorrichtung (10) oder die Bewertungsvorrichtung (20) einen Fehler verursacht, die Steuervorrichtung (30) eine voreingestellte Vorrichtungs-Fehleroperation ausführt, bei der die Steuervorrichtung (30) vorzugsweise einen Warnvorgang als Vorrichtung-Fehleroperation ausführt.

13. Bewertungssystem für biologische Proben nach Anspruch 12,
bei dem die Steuervorrichtung (30) die Verarbeitungsvorrichtung (10) und die Bewertungsvorrichtung (10) als Vorrichtungs-Fehleroperation anhält.

14. Bewertungssystem für biologische Proben nach Anspruch 11 oder 12,
bei dem für den Fall, dass entweder die Verarbeitungsvorrichtung (10) oder die Bewertungsvorrichtung (20) einen Fehler verursacht, die Steuervorrichtung (30) eine Vorrichtung anhält und die andere Vorrichtung kontinuierlich weiterbetreibt.

15. Bewertungsverfahren für biologische Proben, umfassend:
Veranlassen einer Verarbeitungsvorrichtung (10), sequentielle Prozesse an einer Mehrzahl biologischer Proben identischen Typs ab einer Referenzzeit auszuführen;
Veranlassen einer Bewertungsvorrichtung (20), sequentiell die mehreren biologischen Proben ab einem Zeitpunkt zu bewerten, zu dem eine vorbestimmte Zeit ausgehend von der Referenzzeit verstreicht, um mehrere Bewertungsergebnisse in einem gewünschten Bewertungszeitintervall (x) zu gewinnen, wobei ein Zeitintervall (t1) jedes Prozesses an jeder der biologischen Proben eingestellt wird auf ein Zeitintervall gleich oder größer als eine kürzeste Prozessbetriebszeit (ta) der Verarbeitungsvorrichtung (10), und ein Zeitintervall (t2) jede Bewertung jeder der biologischen Proben auf ein Zeitintervall eingestellt wird, welches gleich oder größer ist als eine kürzeste Bewertungsbetriebszeit (tb) der Bewertungsvorrichtung (20), und gewonnen wird durch Addieren oder Subtrahieren des gewünschten Bewertungszeitintervalls (x) auf oder von dem Zeitintervall (t1) jedes Prozesses, wobei das gewünschte Bewertungszeitintervall (x) kürzer ist als die kürzeste Bewertungsbetriebszeit (tb),
sequentielles Ausführen von Prozessen an den jeweiligen biologischen Proben in dem Zeitintervall für jeden Prozess ausgehend von einer Referenzzahl (t1), und
sequentielles Bewerten der jeweiligen biologischen Proben in dem Zeitintervall jeder Bewertung von einem Zeitpunkt aus, zu dem eine voreingestellte Zeit seit einer Referenzzeit verstreicht, um mehrere Bewertungsergebnisse in dem gewünschten Bewertungszeitintervall zu erhalten.

16. Nichtflüchtiges, computer-lesbares Aufzeichnungsmedium, das ein Bewertungssteuerprogramm für biologische Proben speichert, um eine Funktion einer Steuervorrichtung (30) im Rahmen eines Bewertungssystems für biologische Proben zu realisieren, welches eine Verarbeitungsvorrichtung (10) aufweist, die einen identischen Prozess an einer Mehrzahl biologischer Proben identischen Typs ausführt, eine Bewertungsvorrichtung (20) aufweist, die die mehreren biologischen Proben bewertet, und die Steuervorrichtung (30) enthält, welche ein Zeitintervall (t1) jedes Prozesses an jeder der biologischen Proben in der Verarbeitungsvorrichtung (10) steuert, und ein Zeitintervall (t1) jede Bewertung jeder biologischen Probe in der Bewertungsvorrichtung (20) steuert, wobei das Programm die Steuervorrichtung (30) veranlasst, Folgendes auszuführen:
eine Prozedur des Einstellens des Zeitintervalls (t1) jedes Prozesses auf ein Zeitintervall, welches gleich oder größer ist als eine kürzeste Prozessbetriebszeit (ta) der Verarbeitungsvorrichtung (10);
eine Prozedur des Einstellens des Zeitintervalls (t2) jede Bewertung auf ein Zeitintervall, welches gleich oder größer ist als eine kürzeste Bewertungsbetriebszeit (tb) der Bewertungsvorrichtung (20), und gewonnen wird durch Addieren und Subtrahieren eines gewünschten Bewertungszeitintervalls (x), welches kürzer ist als die kürzeste Bewertungsbetriebszeit (tb) auf oder von dem Zeitintervall (t1) jedes Prozesses;
eine Prozedur des sequentiellen Ausführens von Prozessen an den jeweiligen biologischen Proben in dem Zeitintervall (t1) jedes Prozesses ab einem Referenzzeitpunkt; und
eine Prozedur des sequentiellen Bewertens der jeweiligen biologischen Proben in dem Zeitintervall (t2) jeder Auswertung von einem Zeitpunkt an, zu dem eine voreingestellte Zeit ab einer Referenzzeit verstreicht, um mehrere Bewertungsergebnisse in dem gewünschten Bewertungszeitintervall (x) zu erhalten.

## Revendications

1. Système d'évaluation d'échantillons biologiques, comprenant un appareil de traitement (10), un appareil d'évaluation (20), et un appareil de commande (30), lequel commande l'appareil de traitement (10) et l'appareil d'évaluation (20),
**caractérisé en ce que** :
l'appareil de traitement (10) réalise un traitement identique sur une pluralité d'échantillons biologiques d'un type identique ;
l'appareil d'évaluation (20) évalue la pluralité d'échantillons biologiques, et
l'appareil de commande (30), lequel commande un intervalle de temps (t1) de chaque traitement sur chacun des échantillons biologiques dans l'appareil de traitement (10) et un intervalle de temps (t2) de chaque évaluation de chaque échantillon biologique dans l'appareil d'évaluation (20),
dans lequel l'appareil de commande (30) règle l'intervalle de temps (t1) de chaque traitement sur un intervalle de temps, lequel est supérieur ou égal à un temps d'opération de traitement le plus court (ta) de l'appareil de traitement (10), règle l'intervalle de temps (t2) de chaque évaluation sur un intervalle de temps, lequel est supérieur ou égal à un temps d'opération d'évaluation le plus court (tb) de l'appareil d'évaluation (20), et est obtenu en additionnant ou soustrayant un intervalle de temps d'évaluation souhaité plus court que le temps d'opération d'évaluation le plus court (tb) avec l'intervalle de temps (t1) de chaque traitement ;
l'appareil de traitement (10) réalise (S20) de manière séquentielle des traitements sur des échantillons biologiques respectifs avec l'intervalle de temps (t1) entre chaque traitement à partir d'un temps de référence, et
l'appareil d'évaluation (20) évalue (S22) de manière séquentielle les échantillons biologiques respectifs avec l'intervalle de temps entre chaque évaluation de chaque échantillon biologique à partir d'un point dans le temps auquel un temps préréglé s'écoule à partir du temps de référence, afin d'obtenir une pluralité de résultats d'évaluation sur l'intervalle de temps souhaité.

2. Système d'évaluation d'échantillons biologiques selon la revendication 1, dans lequel dans un cas où le temps d'opération de traitement le plus court (ta) est inférieur ou égal au temps d'opération d'évaluation le plus court (tb) (S14 : OUI) et où l'intervalle de temps d'évaluation souhaité (x) est inférieur ou égal à la valeur du temps d'opération d'évaluation le plus court (tb) moins le temps d'opération de traitement le plus court (ta) (S26 : OUI), l'appareil de commande (30) règle l'intervalle de temps (t1) de chaque traitement sur la valeur du temps d'opération d'évaluation le plus court (tb) moins l'intervalle de temps d'évaluation souhaité (x), et règle l'intervalle de temps (t2) de chaque évaluation de chaque échantillon biologique sur le temps d'opération d'évaluation le plus court (tb) (S18), et
dans un cas où le temps d'opération de traitement le plus court (ta) est supérieur au temps d'opération d'évaluation le plus court (tb) (S14 : NON) et où l'intervalle de temps d'évaluation souhaité (x) est inférieur ou égal à la valeur du temps d'opération de traitement le plus court (ta) moins le temps d'opération d'évaluation le plus court (tb) (S26 : OUI), l'appareil de commande (30) règle l'intervalle de temps (t1) de chaque traitement sur la valeur du temps d'opération de traitement le plus court (ta), et règle l'intervalle de temps (t2) de chaque évaluation de chaque échantillon biologique sur la valeur du temps d'opération de traitement le plus court (ta) moins l'intervalle de temps d'évaluation souhaité (x) (S28).

3. Système d'évaluation d'échantillons biologiques selon la revendication 1,
dans lequel l'appareil de commande (30) acquiert (S10) le temps d'opération de traitement le plus court (ta) et le temps d'opération d'évaluation le plus court (tb), et règle l'intervalle de temps (t1) de chaque traitement et l'intervalle de temps (t2) de chaque évaluation sur la base du temps d'opération de traitement le plus court (ta) et du temps d'opération d'évaluation le plus court (tb) acquis, de l'intervalle de temps souhaité (x) et du temps écoulé T à partir du temps de référence.

4. Système d'évaluation d'échantillons biologiques selon la revendication 3,
dans lequel l'appareil de commande (30) présente un mode opérationnel dans lequel l'intervalle de temps (t1) de chaque traitement et l'intervalle de temps (t2) de chaque évaluation sont réglés de telle sorte qu'un temps requis pour terminer tous les traitements et évaluations pour la pluralité d'échantillons biologiques est le plus court.

5. Système d'évaluation d'échantillons biologiques selon la revendication 3 ou 4,
dans lequel l'appareil de commande (30) présente un mode opérationnel dans lequel l'intervalle de temps (t1) de chaque traitement et l'intervalle de temps (t2) de chaque évaluation sont réglés de telle sorte qu'un temps requis pour terminer tous les traitements et évaluations pour la pluralité d'échantillons biologiques est un temps quelconque désigné à l'avance.

6. Système d'évaluation d'échantillons biologiques selon l'une quelconque des revendications 1 à 5,
dans lequel l'appareil de commande (30) présente un mode opérationnel dans lequel l'intervalle de temps (t1) de chaque traitement et l'intervalle de temps (t2) de chaque évaluation sont réglés de telle sorte qu'un temps requis pour le traitement et l'évaluation pour un seul échantillon biologique est inférieur ou égal à un temps préréglé.

7. Système d'évaluation d'échantillons biologiques selon l'une quelconque des revendications 1 à 6,
dans lequel l'appareil de commande (30) présente un mode opérationnel dans lequel l'intervalle de temps (t1) de chaque traitement et l'intervalle de temps (t2) de chaque évaluation sont réglés de telle sorte qu'un temps d'utilisation total de l'appareil de traitement (10) est inférieur ou égal à un temps préréglé.

8. Système d'évaluation d'échantillons biologiques selon l'une quelconque des revendications 1 à 7,
dans lequel l'appareil de commande (30) présente un mode opérationnel dans lequel l'intervalle de temps (t1) de chaque traitement et l'intervalle de temps (t2) de chaque évaluation sont réglés de telle sorte qu'un temps d'utilisation total de l'appareil d'évaluation (20) est inférieur ou égal à un temps préréglé.

9. Système d'évaluation d'échantillons biologiques selon l'une quelconque des revendications 5 à 8,
dans lequel dans un cas où l'appareil de commande (30) n'est pas en mesure de déterminer l'intervalle de temps (t1) de chaque traitement et l'intervalle de temps (t2) de chaque évaluation satisfaisant une condition du mode opérationnel, l'appareil de commande (30) réalise une opération d'erreur de temporisation préréglée.

10. Système d'évaluation d'échantillons biologiques selon la revendication 9,
dans lequel l'appareil de commande (30) réalise une opération d'avertissement comme opération d'erreur de temporisation.

11. Système d'évaluation d'échantillons biologiques selon la revendication 9 ou 10,
dans lequel comme opération d'erreur de temporisation, l'appareil de commande (30) affiche l'intervalle de temps (t1) de chaque traitement et l'intervalle de temps (t2) de chaque évaluation, lesquels sont réalisables sur un appareil d'affichage (50).

12. Système d'évaluation d'échantillons biologiques selon l'une quelconque des revendications 1 à 11,
dans lequel dans un cas où l'appareil de traitement (10) ou l'appareil d'évaluation (20) provoque une erreur, l'appareil de commande (30) réalise une opération d'erreur d'appareil préréglée, dans lequel de préférence, l'appareil de commande (30) réalise une opération d'avertissement comme opération d'erreur d'appareil.

13. Système d'évaluation d'échantillons biologiques selon la revendication 12,
dans lequel l'appareil de commande (30) arrête l'appareil de traitement (10) et l'appareil d'évaluation (20) comme opération d'erreur d'appareil.

14. Système d'évaluation d'échantillons biologiques selon la revendication 11 ou 12,
dans lequel dans un cas où soit l'appareil de traitement (10), soit l'appareil d'évaluation (20) provoque une erreur, l'appareil de commande (30) arrête un appareil, et fait fonctionner de manière continue l'autre appareil.

15. Procédé d'évaluation d'échantillons biologiques, comprenant les étapes consistant à :
faire en sorte qu'un appareil de traitement (10) réalise de manière séquentielle des traitements sur une pluralité d'échantillons biologiques d'un type identique à partir d'un temps de référence ;
faire en sorte qu'un appareil d'évaluation (20) évalue de manière séquentielle la pluralité d'échantillons biologiques à partir d'un point dans le temps auquel un temps préréglé s'écoule à partir du temps de référence, afin d'obtenir une pluralité de résultats d'évaluation sur un intervalle de temps d'évaluation souhaité (x), dans lequel un intervalle de temps (t1) de chaque traitement sur chacun des échantillons biologiques est réglé sur un intervalle de temps, lequel est supérieur ou égal à un temps d'opération de traitement le plus court (ta) de l'appareil de traitement (10), et un intervalle de temps (t2) de chaque évaluation de chacun des échantillons biologiques est réglé sur un intervalle de temps, lequel est supérieur ou égal à un temps d'opération d'évaluation le plus court (tb) de l'appareil d'évaluation (20), et est obtenu en additionnant ou soustrayant intervalle de temps d'évaluation souhaité (x) avec l'intervalle de temps (t1) de chaque traitement, l'intervalle de temps d'évaluation souhaité (x) étant plus court que le temps d'opération d'évaluation le plus court (tb) ;
réaliser de manière séquentielle des traitements sur les échantillons biologiques respectifs sur l'intervalle de temps de chaque traitement à partir d'un temps de référence (t1), et
évaluer de manière séquentielle les échantillons biologiques respectifs sur l'intervalle de temps de chaque évaluation à partir d'un point dans le temps auquel un temps préréglé s'écoule à partir du temps de référence, afin d'obtenir une pluralité de résultats d'évaluation sur l'intervalle de temps d'évaluation souhaité.

16. Support d'enregistrement lisible par ordinateur non transitoire stockant un programme de commande d'évaluation d'échantillons biologiques pour réaliser une fonction d'un appareil de commande (30) dans un système d'évaluation d'échantillons biologiques comprenant un appareil de traitement (10), lequel réalise un traitement identique sur une pluralité d'échantillons biologiques d'un type identique ; un appareil d'évaluation (20), lequel évalue la pluralité d'échantillons biologiques, et l'appareil de commande (30), lequel commande un intervalle de temps (t1) de chaque traitement sur chacun des échantillons biologiques dans l'appareil de traitement (10) et un intervalle de temps (t2) de chaque évaluation de chaque échantillon biologique dans l'appareil d'évaluation (20), le programme faisant en sorte que l'appareil de commande (30) exécute :
une procédure pour régler l'intervalle de temps (t1) de chaque traitement sur un intervalle de temps, lequel est supérieur ou égal à un temps d'opération de traitement le plus court (ta) de l'appareil de traitement (10) ;
une procédure pour régler l'intervalle de temps (t2) de chaque évaluation sur un intervalle de temps, lequel est supérieur ou égal au temps d'opération d'évaluation le plus court (tb) de l'appareil d'évaluation (20), et est obtenu en additionnant ou soustrayant un intervalle de temps d'évaluation souhaité (x), lequel est plus court que le temps d'opération d'évaluation le plus court (tb) avec l'intervalle de temps (t1) de chaque traitement,
une procédure pour réaliser de manière séquentielle des traitements sur les échantillons biologiques respectifs sur l'intervalle de temps (t1) entre chaque traitement à partir d'un temps de référence, et
une procédure pour évaluer de manière séquentielle les échantillons biologiques respectifs sur l'intervalle de temps (t2) de chaque évaluation à partir d'un point dans le temps auquel un temps préréglé s'écoule à partir du temps de référence, afin d'obtenir une pluralité de résultats d'évaluation sur l'intervalle de temps d'évaluation souhaité (x).
